# EUROPEAN PATENT SPECIFICATION

(11) **EP 4 140 397 B1**
(45) Date of publication and mention of the grant of the patent: **02.09.2026**
(21) Application number: 22188229.3
(22) Date of filing: 02.08.2022
(51) Int. Cl.: A61B 5/00, A61B 5/0205, A61B 5/08, A61B 5/11, A61B 5/113, A61B 5/1455

(54) **EAR-WEARABLE ELECTRONIC DEVICE INCLUDING IN-EAR RESPIRATION RATE SENSOR**
AM OHR TRAGBARE ELEKTRONISCHE VORRICHTUNG MIT EINEM SENSOR IM OHR ZUR MESSUNG DER ATEMFREQUENZ
DISPOSITIF ÉLECTRONIQUE À PORTER À L'OREILLE COMPRENANT UN CAPTEUR DE FRÉQUENCE RESPIRATOIRE INTRA-AURICULAIRE

(30) Priority: 31.08.2021 US 202163238950 P
(43) Date of publication of application: 01.03.2023
(73) Proprietor: Starkey Laboratories, Inc., Eden Prairie, MN 55344 (US)
(72) Inventor: Talman, Roy, Tel Aviv (IL); Ganor, Yaniv, Tel Aviv (IL); Weizman, Lior, Tel Aviv (IL); Aviel, Neta, Tel Aviv (IL); Bornstein, Nitzan, Tenafly (US)
(74) Representative: Vossius & Partner Patentanwälte Rechtsanwälte mbB

(56) References cited:
- WO-A2-2020/146248
- US-A1- 2019 142 625
- US-A1- 2021 000 384

## Description

### TECHNICAL FIELD

This application relates generally to devices and sensing methods for measuring respiration rate from within an ear, such devices including ear-wearable electronic devices, hearing aids, commercial hearables, earbuds, personal amplification devices, and physiologic/biometric monitoring devices.

### BACKGROUND

Respiration rate (RR), referred to interchangeably as respiratory rate, is an important vital sign to check when assessing the health of a patient. This vital sign provides information on clinical deterioration of a patient, is predictive of cardiac arrest, and supports the diagnosis of severe pneumonia, for example. Respiration rate responds to a variety of stressors, including stress, cognitive load, cold, and hyperthermia, for example. While exercising, respiration rate can serve as a good marker of physical effort and fatigue.

WO 2020/146248 A2 discloses methods and apparatuses for estimating a biometric parameter using multiple sensors. Multiple sensors are strategically used to output with increased confidence a biometric parameter. Multiple sensors are strategically used to save power while determining an accurate biometric estimation. Additionally, multiple sensors are used to detect and categorize a sleep apnea event.

US 2019/0142625 A1 discloses an obstructive sleep apnea detection device including an optical engagement surface adapted to engage a user's skin; a light source adapted to emit light from the optical engagement surface; a photodetector adapted to detect light at the optical engagement surface and to generate a detected light signal; a position sensor adapted to determine patient sleeping position; a controller adapted to determine and record in memory blood oxygen saturation values computed from the detected light signal and user position information from the position sensor; and a housing supporting the optical engagement surface, the photodetector, the light source, the position sensor, and the controller.

### SUMMARY

The claimed invention is defined by the appended independent claims 1 and 13. Further embodiments of the claimed invention are described in the appended dependent claims.
The claimed invention is directed to a method of determining respiration rate using an ear-wearable electronic device as defined by the appended independent claim 1. The method involves obtaining motion information from a motion sensor of the ear-wearable electronic device, and generating a first respiration rate estimate using the motion information. The method also involves obtaining photoplethysmographic (PPG) data from a PPG sensor of the ear-wearable electronic device, and generating a second respiration rate estimate using the PPG data. The method further involves producing a respiration rate estimate using the first and second respiration rate estimates, performing an activity status test using the motion information and PPG data; and determining if a measure of the wearer's physical activity based on the motion information is consistent with the wearer's respiration rate estimated using the PPG data.

The claimed invention is further directed to an ear-wearable electronic device as defined by the appended independent claim 13. The device comprises a motion sensor configured to generate motion information and information processing circuitry configured to generate a first respiration rate estimate using the motion information. A PPG sensor is configured to generate PPG data and PPG data processing circuitry is configured to generate a second respiration rate estimate using the PPG data. A processor is configured to produce a respiration rate estimate using the first and second respiration rate estimates, to perform an activity status test using the motion information and PPG data, and to determine if a measure of the wearer's physical activity based on the motion information is consistent with the wearer's respiration rate estimated using the PPG data. The claimed ear-wearable electronic device may further comprise a communication device that is configured to communicate the respiration rate estimate to one or both of an external electronic device and a cloud database.

Also disclosed is a method of determining respiration rate using an ear-wearable electronic device comprising obtaining motion information indicative of body motion from a motion sensor of the ear-wearable electronic device, generating, using a processor of the device, a respiration rate estimate using the motion information, and communicating the respiration rate estimate to one or both of an external electronic device and a cloud database.

Further disclosed is an ear-wearable electronic device comprising a motion sensor configured to generate motion information indicative of body motion, a processor configured to produce a respiration rate estimate using the motion information, and a communication device configured to communicate the respiration rate estimate to one or both of an external electronic device and a cloud database.

Further disclosed yet is a method of determining respiration rate using an ear-wearable electronic device comprising obtaining photoplethysmographic (PPG) data from a PPG sensor of the ear-wearable electronic device, generating, using a processor of the device, a respiration rate estimate using the PPG data, and communicating the respiration rate estimate to one or both of an external electronic device and a cloud database.

Also disclosed is an ear-wearable electronic device comprising a photoplethysmographic (PPG) sensor configured to generate PPG data, a processor configured to produce a respiration rate estimate using the PPG data, and a communication device configured to communicate the respiration rate estimate to one or both of an external electronic device and a cloud database.

The above summary is not intended to describe each disclosed embodiment or every implementation of the present disclosure. The figures and the detailed description below more particularly exemplify illustrative embodiments.

### BRIEF DESCRIPTION OF THE DRAWINGS

Throughout the specification reference is made to the appended drawings wherein:
Figure 1 is a block diagram of a representative ear-wearable electronic device which includes a respiration rate sensor in accordance with any of the embodiments disclosed herein;
Figure 2 illustrates a respiration sensor integral to an ear-wearable electronic device in accordance with any of the embodiments disclosed herein;
Figure 3 illustrates a method implemented by the ear-wearable electronic device illustrated in Figure 2;
Figure 4 illustrates a respiration sensor deployed in an ear-wearable electronic device in accordance with any of the embodiments disclosed herein;
Figure 5 illustrates a respiration sensor integral to an ear-wearable electronic device in accordance with any of the embodiments disclosed herein;
Figure 6 illustrates a method implemented by an ear-wearable electronic device illustrated in Figure 5;
Figure 7 illustrates a respiration sensor deployed in an ear-wearable electronic device in accordance with any of the embodiments disclosed herein;
Figure 8 illustrates a respiration sensor deployed in an ear-wearable electronic device in accordance with any of the embodiments disclosed herein;
Figure 9 illustrates a respiration sensor deployed in an ear-wearable electronic device in accordance with any of the embodiments disclosed herein;
Figure 10 illustrates additional processing details implemented by the PPG sensing circuitry and the motion sensing circuitry shown in previous figures;
Figure 11 illustrates a method of selecting the best window of time from a sampling of PPG data and/or motion sensor data in accordance with any of the embodiments disclosed herein;
Figure 12 illustrates a method of applying a sinus-fitting algorithm on PPG data and/or motion sensor data in accordance with any of the embodiments disclosed herein;
Figure 13 illustrates a method for activating a respiration rate sensor of an ear-wearable electronic device in accordance with any of the embodiments disclosed herein; and
Figure 14 illustrates a system involving the generation and distribution of respiration rate data by an ear-wearable electronic device in accordance with any of the embodiments disclosed herein.

The figures are not necessarily to scale. Like numbers used in the figures refer to like components. However, it will be understood that the use of a number to refer to a component in a given figure is not intended to limit the component in another figure labeled with the same number.

### DETAILED DESCRIPTION

Valuable health information can be derived from measurement of a person's respiration rate. Provision of a respiration rate sensing facility in an ear-wearable electronic device can improve the accuracy of disease detection as well as open new doors to many more feature possibilities, including emotional stress monitoring and cardiac arrythmia detection. Additionally, an ear-wearable electronic device which incorporates a respiration rate sensing facility according to the present disclosure can assist in tracking the progression of an illness, such as respiratory diseases. By tracking respiration rate and detecting increases in respiration rate overtime (e.g., via trend analyses), the worsening of respiratory disease can be accurately detected and monitored.

Some embodiments are directed to an ear-wearable electronic device which incorporates a PPG-based sensing facility configured to generate an estimate of a wearer's respiration rate. Some embodiments are directed to an ear-wearable electronic device which incorporates a motion-based sensing facility configured to generate an estimate of the wearer's respiration rate. Some embodiments are directed to an ear-wearable electronic device which incorporates a PPG-based sensing facility and a motion-based sensing facility configured to generate an estimate of the wearer's respiration rate.

Respiration induces variations in a photoplethysmogram in three different ways. (1) Respiratory-Induced Intensity Variation (RIIV): involves changes in venous return due to changes in intra-thoracic pressure throughout the respiratory cycle, which cause a baseline (DC) modulation of the PPG signal. (2) Respiratory-Induced Amplitude Variation (RIAV): during inspiration, left ventricular stroke volume decreases due to changes in intra-thoracic pressure leading to the decreased pulse amplitude. The opposite occurs during expiration. (3) Respiratory-Induced Frequency Variation (RIFV): heart rate varies throughout the respiratory cycle. Heart rate increases during inspiration and decreases during expiration. This phenomenon, known as respiratory sinus arrhythmia (RSA), is mainly due to the autonomic regulation of HR during respiration.

Respiration induces variations in a motion sensor signal. During respiration, the body moves according to the breathing rate of the subject. For example, the chest wall expands and contracts in response to inhalation and exhalation during breathing. By analyzing these motions, measured by a motions sensor (e.g., an accelerometer), respiration rate can be estimated.

Embodiments of the disclosure are defined in the claims.

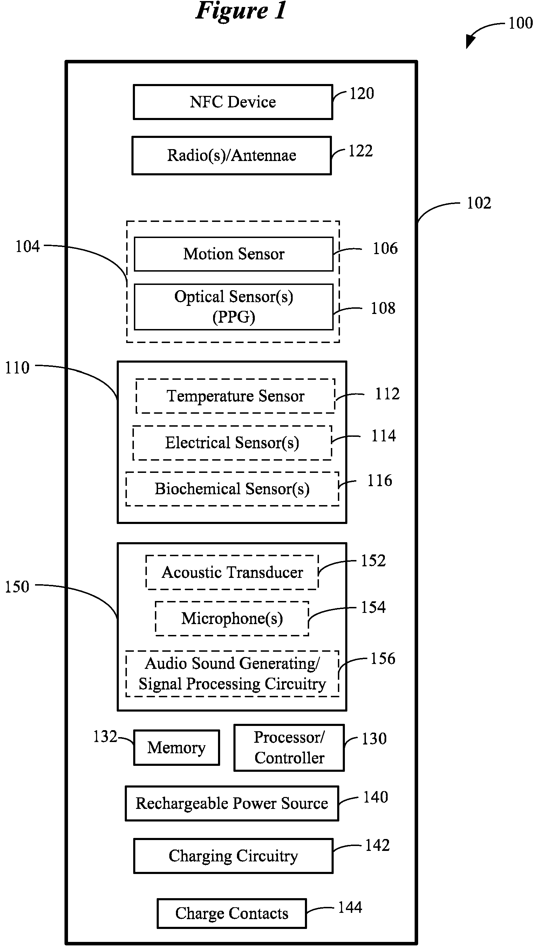

Figure 1 is a block diagram of a representative ear-wearable electronic device 100 which incorporates a respiration sensor in accordance with any of the embodiments disclosed herein. The device 100 is representative of a wide variety of electronic devices configured to be deployed in, on or about an ear of a wearer, including any of the devices discussed herein. **In** some implementations, the device 100 is configured as a hearable in which amplified sound is communicated one or both ears of a wearer. **In** other implementations, the device 100 is configured as a physiologic or biometric monitoring device, which may include or exclude sound amplification circuitry. The device 100 can be configured to include or exclude some of the representative components shown in Figure 1.

The term ear-wearable electronic device 100 of the present disclosure refers to a wide variety of ear-wearable electronic devices that can aid a person with impaired hearing. The term ear-wearable electronic device also refers to a wide variety of devices that can produce optimized or processed sound for persons with normal hearing. Ear-wearable electronic devices of the present disclosure include hearables (e.g., earbuds) and hearing aids (e.g., hearing instruments), for example. Ear-wearable electronic devices include, but are not limited to ITE, ITC, CIC or IIC type hearing devices or some combination of the above. Some ear-wearable electronic devices can be devoid of an audio processing facility, and be configured as an ear-wearable biometric sensor (e.g., a respiration sensor alone or in combination with any of the other physiologic and/or motion sensors disclosed herein). In this disclosure, reference is made to an "ear-wearable electronic device," which is understood to refer to a system comprising a single ear device (left or right) or both a left ear device and a right ear device.

According to any of the embodiments disclosed herein, the device 100 includes a housing 102 configured for deployment in an ear canal of a wearer. In some implementations, the housing 102 includes a shell having a uniquely-shaped outer surface (e.g., an organic shape) that corresponds uniquely to an ear geometry of a wearer of the device (e.g., a custom RIC in-ear device). In other implementations, the shell of the housing 102 has a generic or standard shape having an outer surface configured for deployment in ear canals of a population of wearers.

The device 100 includes a respiration sensor 104 configured to sense physiologic activity from which a respiration rate estimate is calculated by the device 100. In some implementations, the respiration sensor 104 includes a motion sensor 106, such as an accelerometer (e.g., a 3-axis accelerometer). In other implementations, the respiration sensor 104 includes one or more optical sensors 108, such as a PPG sensor. In some implementations, the respiration sensor 104 includes a motion sensor 106 and a PPG sensor.

In implementations in which the respiration sensor 104 includes a PPG sensor 108, the shell of the housing 102 includes a window through a proximal portion of the shell and positioned at a tragal wall when the device is deployed in the wearer's ear. The PPG sensor 108 is situated within the window of the shell. The shell can include a seal arrangement comprising an acoustic seal to inhibit ambient sound from reaching the wearer's eardrum. The seal arrangement can also include a light seal configured to inhibit ambient light from reaching the PPG sensor 108. The PPG sensor 108 is preferably situated in the window such that no air gaps exist between the PPG sensor 108 and tissue of the ear canal when deployed in the wearer's ear. The combination of a light seal and prevention of air gaps provides for signals produced by the PPG sensor 108 having a high signal-to-noise ratio.

In some implementations (e.g., physiologic or biometric monitoring configurations), the device 100 can include a physiologic sensor facility 110 which can include one or more additional physiologic sensors. For example, the device 100 can include one or more temperature sensors 112 configured to produce signals from which an estimate of a wearer's core body temperature can be calculated by the device 100. The temperature sensor or sensors (e.g., a proximal temperature sensor and a distal temperature sensor) can be thermistors. Various types of thermistors can be incorporated into the ear-wearable electronic devices of the present disclosure (e.g., those having a negative temperature coefficient (e.g., a negative temperature coefficient (NTC) chip) and those having a positive temperature coefficient (PTC)). In some implementations, the temperature sensor(s) 112 can be implemented as a SMD (surface mount device) thermistor, a thermocouple, a resistance temperature detector (RTD), a digital thermistor, or other type of resistance temperature sensors.

The device 100 can include one or more physiologic electrode-based sensors 114, such as an ECG, oxygen saturation (SpO2), respiration, EMG, EEG, EOG, galvanic skin response, and/or electrodermal activity sensor. The device 100 can include one or more biochemical sensors 116 (e.g., glucose concentration, PH value, Ca+ concentration, hydration). Embodiments disclosed herein can incorporate one or more of the sensors disclosed in commonly-owned co-pending U.S. Patent Application Serial Nos. 63/125,700 filed December 15, 2020 under Attorney Docket No. ST0922PRV/0532.000922US60 and 63/126,426 filed December 16, 2020 under Attorney Docket No. ST0931PRV/0532.000931US60.

The device 100 can include an NFC device 120 (e.g., a NFMI device), and, additionally or alternatively, may include one or more RF radios/antennae 122 (e.g., compliant with a Bluetooth^{®} or IEEE 802.11 protocol). The RF radios/antennae 122 can be configured to effect communications with an external electronic device, communication system, and/or the cloud. Data acquired by the ear-wearable electronic device 100 can be communicated to an external electronic device, such as a smartphone, laptop, network server, and/or the cloud (e.g., a cloud server/database and/or processor). The device 100 typically includes a rechargeable power source 140 (e.g., a lithium-ion battery) operably coupled to charging circuitry 142 and charge contacts 144.

The device 100 includes a processor (e.g., a controller) 130 coupled to memory 132. Among other duties, the processor 130 is configured to calculate an estimated respiration rate of the device wearer in accordance with any of the embodiments disclosed herein. The processor 130 is also configured to calculate other biometric or physiologic parameters or conditions using the various sensor signals discussed herein.

**In** accordance with any of the embodiments disclosed herein, the device 100 can be configured as a hearing device or a hearable which includes an audio processing facility 150. The audio processing facility 150 includes sound generating circuitry and can also include audio signal processing circuitry 156 coupled to an acoustic transducer 152 (e.g., a sound generator, speaker, receiver, bone conduction device). In some implementations, the audio processing facility 150 includes one or more microphones 154 coupled to the audio signal processing circuitry 156. In other implementations, the device 100 can be devoid of the one or more microphones 154. In further implementations, the device 100 can be devoid of the audio processing facility 150, and be configured as an ear-wearable biometric sensor (e.g., a respiration sensor alone or in combination with any of the other sensors disclosed herein).

According to implementations that incorporate the audio processing facility 150, the device 100 can be implemented as a hearing assistance device that can aid a person with impaired hearing. For example, the device 100 can be implemented as a monaural hearing aid or a pair of devices 100 can be implemented as a binaural hearing aid system, in which case left and right devices 100 are deployable with corresponding left and right wearable sensor units. The monaural device 100 or a pair of devices 100 can be configured to effect bi-directional communication (e.g., wireless communication) of data with an external source, such as a remote server via the Internet or other communication infrastructure. The device or devices 100 can be configured to receive streaming audio (e.g., digital audio data or files) from an electronic or digital source. Representative electronic/digital sources (e.g., accessory devices) include an assistive listening system, a streaming device (e.g., a TV streamer or audio streamer), a remote microphone, a radio, a smartphone, a laptop, a cell phone/entertainment device or other electronic device that serves as a source of digital audio data, control and/or settings data or commands, and/or other types of data files.

The processor/controller 130 shown in Figure 1 can include one or more processors or other logic devices. For example, the processor/controller 130 can be representative of any combination of one or more logic devices (e.g., multi-core processor, digital signal processor (DSP), microprocessor, programmable controller, general-purpose processor, special-purpose processor, hardware controller, software controller, a combined hardware and software device) and/or other digital logic circuitry (e.g., ASICs, FPGAs), and software/firmware configured to implement the functionality disclosed herein. The processor/controller 130 can incorporate or be coupled to various analog components (e.g., analog front-end), ADC and DAC components, and Filters (e.g., FIR filter, Kalman filter). The processor/controller 130 can incorporate or be coupled to memory 132 as previously discussed. The memory 132 can include one or more types of memory, including ROM, RAM, SDRAM, NVRAM, EEPROM, and FLASH, for example.

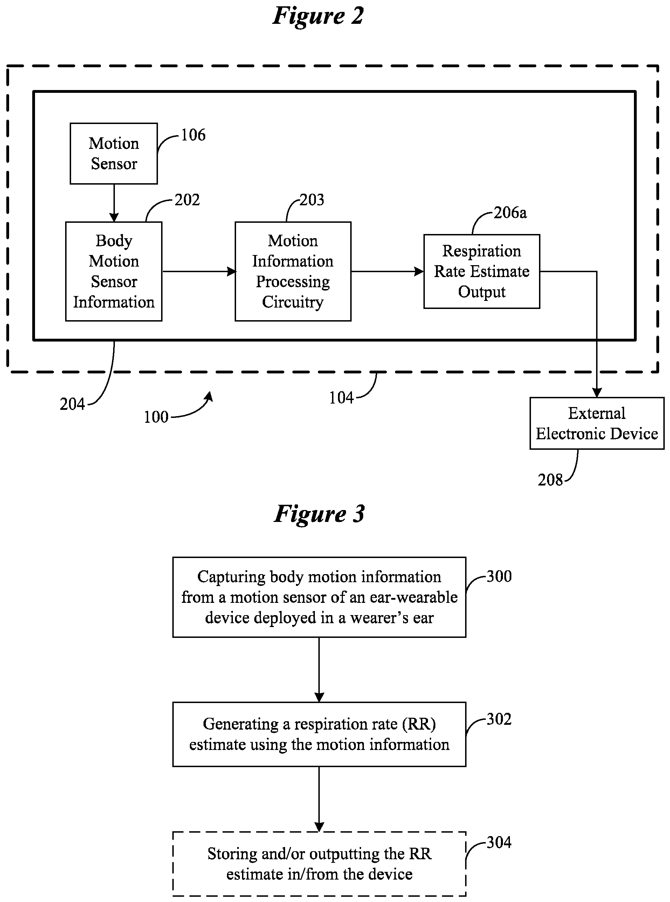

Figure 2 illustrates a respiration sensor 104 integral to an ear-wearable electronic device 100 in accordance with any of the embodiments disclosed herein. The respiration sensor 104 illustrated in Figure 2 includes motion sensing circuitry 204 comprising a motion sensor 106. The motion sensor 106 can be or include an accelerometer (e.g., a 3-axis accelerometer), a gyroscope, an inertial measurement unit (IMU), a magnetometer or any combination of these motion sensing devices. The motion sensor 106 is configured to produce body motion sensor information 202 responsive to movement of a wearer's body. For example, the motion sensor 106 can be configured to be sensitive to chest wall motion associated with inhalation and exhalation during breathing by the wearer. The body motion sensor information 202 is communicated to motion information processing circuitry 203, which may be integral in whole or in part to the processor 130 or to a separate processor or digital logic circuitry. The motion information processing circuitry 203 is configured to generate an estimate of respiration rate using the body motion sensor information 202. The respiration rate estimate is communicated to an output 206a of the motion sensing circuitry 204. The respiration rate estimate output 206a can be communicated to an external electronic device 208 (e.g., via a communication device of the ear-wearable electronic device 100) and/or stored in a memory of the ear-wearable electronic device 100.

Figure 3 illustrates a method implemented by the ear-wearable electronic device 100 illustrated in Figure 2. The method shown in Figure 3 involves capturing 300 body motion information from a motion sensor of an ear-wearable electronic device deployed in the wearer's ear. The method also involves generating 302 a respiration rate estimate using the motion information. The method may also involves storing and/or outputting 304 the respiration rate estimate in/from the ear-wearable electronic device.

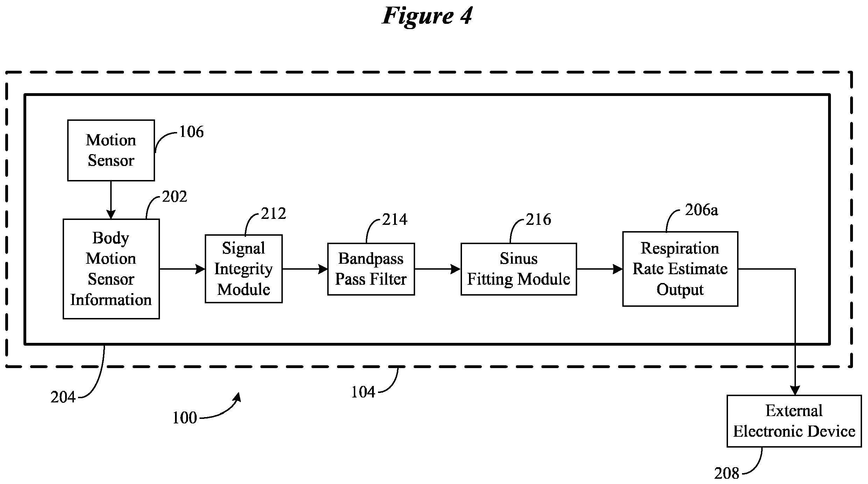

Figure 4 illustrates a respiration sensor 104 deployed in an ear-wearable electronic device 100 in accordance with any of the embodiments disclosed herein. The respiration sensor 104 illustrated in Figure 4 shows additional details of the motion sensing circuitry 204 shown in Figure 3. The motion sensing circuitry 204 includes a motion sensor 106 of a type previously described which is configured to produce body motion sensor information 202 in a manner previously described.

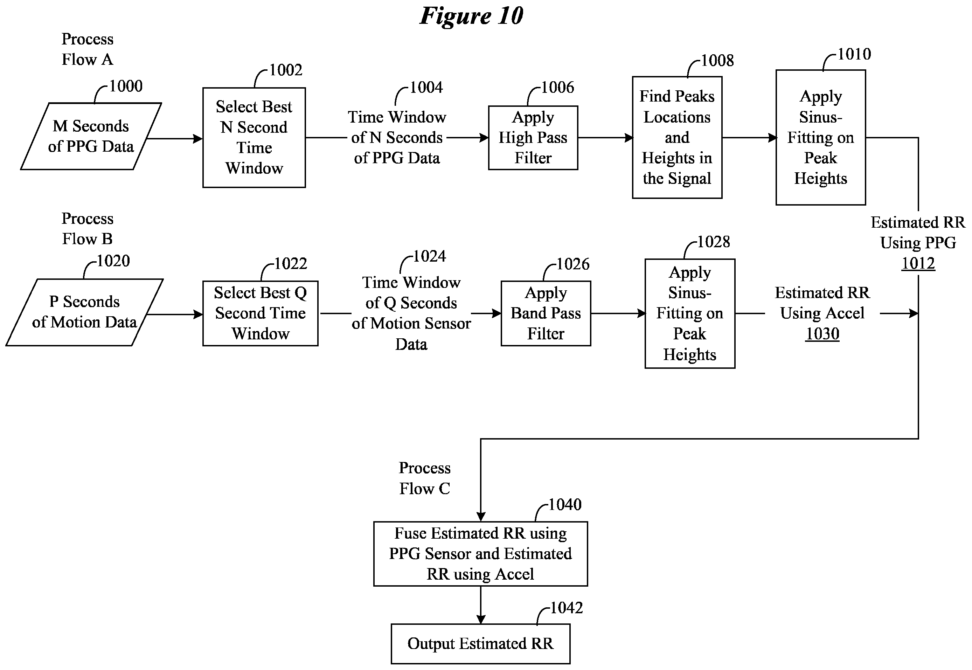

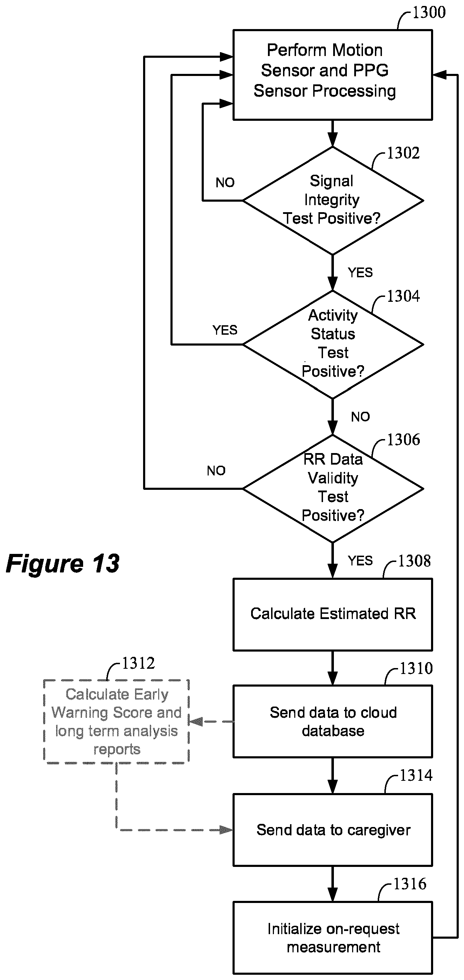

As shown in Figure 4, the body motion sensor information 202 is communicated to a signal integrity module 212 which is configured to perform a signal integrity test using various metrics, details of which will be described with reference to Figure 13. A selected time segment of the body motion sensor information 202 is communicated to a bandpass filter 214 configured to pass sensor information 202 within a frequency range consistent with human breathing. For example, the bandpass filter 214 can be configured to pass frequencies in the range of about 0.15 Hz to about 0.5 Hz. The time segment of the body motion sensor information 202 is selected based on a time window selection algorithm, details of which are shown in Figure 10. The bandpass-filtered body motion sensor information 202 is communicated to a sinus fitting module 216 configured to perform a least-squares fitting operation, details of which will be described with reference to Figure 10. The sinus fitting module 216 generates a respiration rate estimate output 206a which can be stored internally within the ear-wearable electronic device 100 and/or communicated to an external electronic device 208.

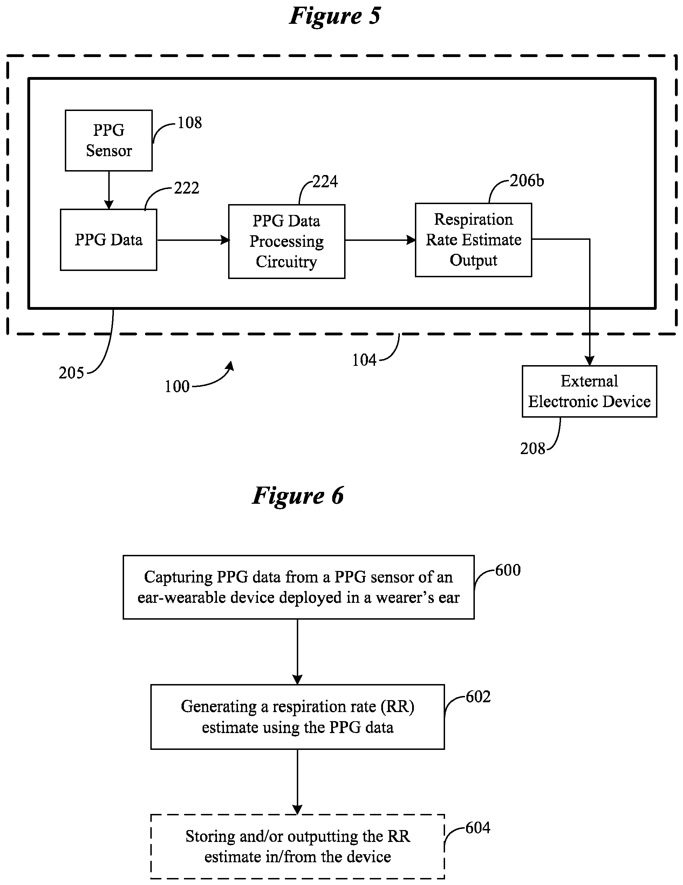

Figure 5 illustrates a respiration sensor 104 integral to an ear-wearable electronic device 100 in accordance with any of the embodiments disclosed herein. The respiration sensor 104 illustrated in Figure 5 includes PPG sensing circuitry 205 comprising a PPG sensor 108. The PPG sensor 108 may be implemented as a pulse oximeter or other form of sensor capable of optically obtaining a plethysmogram. The PPG sensor 108 is configured to produce PPG data 222 from within the wearer's ear. For example, the PPG sensor 108 can be situated in a window of the housing 102 of the ear-wearable electronic device 100 such that the PPG sensor 108 faces the tragal wall when the device 100 is fully deployed in the wearer's ear. As was previously discussed, the shell of the device housing can include a seal arrangement comprising an acoustic seal and a light seal, wherein the light seal is configured to inhibit ambient light from reaching the PPG sensor 108. The PPG sensor is situated in the window of the shell so that no air gaps exist between the PPG sensor 108 and tissue of the wearer's tragal wall when the device 100 is deployed in the wearer's ear.

PPG data 222 produced by the PPG sensor 108 is communicated to PPG data processing circuitry 224, which may be integral in whole or in part to the processor 130 or to a separate processor or digital logic circuitry. The PPG data processing circuitry 224 is configured to generate an estimate of respiration rate using the PPG data 222. The respiration rate estimate is communicated to an output 206b of the PPG sensing circuitry 205. The respiration rate estimate output 206b can be communicated to an external electronic device 208 (e.g., via a communication device of the ear-wearable electronic device 100) and/or stored in a memory 132 of the ear-wearable electronic device 100.

Figure 6 illustrates a method implemented by an ear-wearable electronic device 100 illustrated in Figure 5. The method shown in Figure 6 involves capturing 600 PPG data from a PPG sensor of an ear-wearable electronic device deployed in the wearer's ear. The method also involves generating 603 a respiration rate estimate using the PPG data. The method may also involve storing and/or outputting 604 the respiration rate estimate in/from the ear wearable electronic device.

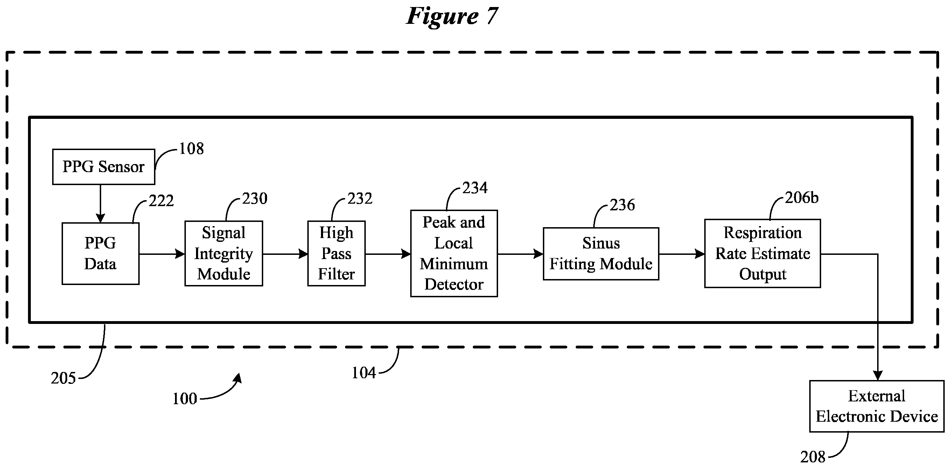

Figure 7 illustrates a respiration sensor 104 deployed in an ear-wearable electronic device 100 in accordance with any of the embodiments disclosed herein. The respiration sensor 104 illustrated in Figure 7 shows additional details of the PPG sensing circuitry 205. The PPG data 222 produced by the PPG sensor 108 is communicated to a signal integrity module 230 which is configured to perform a signal integrity test using various metrics, details of which will be described with reference to Figure 13. A selected time segment of the PPG data 222 is communicated to a high pass filter 232 configured to remove noise that appears in low frequencies that are irrelevant for the respiration rate estimation. For example, the high pass filter 232 can be configured to pass frequencies above about 0.3 Hz (e.g., f_{corner} = 0.3 Hz). The high pass-filtered PPG data 222 is communicated to a peak and local minimum detector 234, which is configured to analyze the amplitude variations of the high pass-filtered PPG data 222. The high pass-filtered PPG data 222 is communicated to a sinus fitting module 236 configured to perform a least-squares fitting operation, details of which will be described with reference to Figure 10. The sinus fitting module 236 generates a respiration rate estimate output 206b which can be stored internally within the memory 132 of the ear-wearable electronic device 100 and/or communicated to an external electronic device 208.

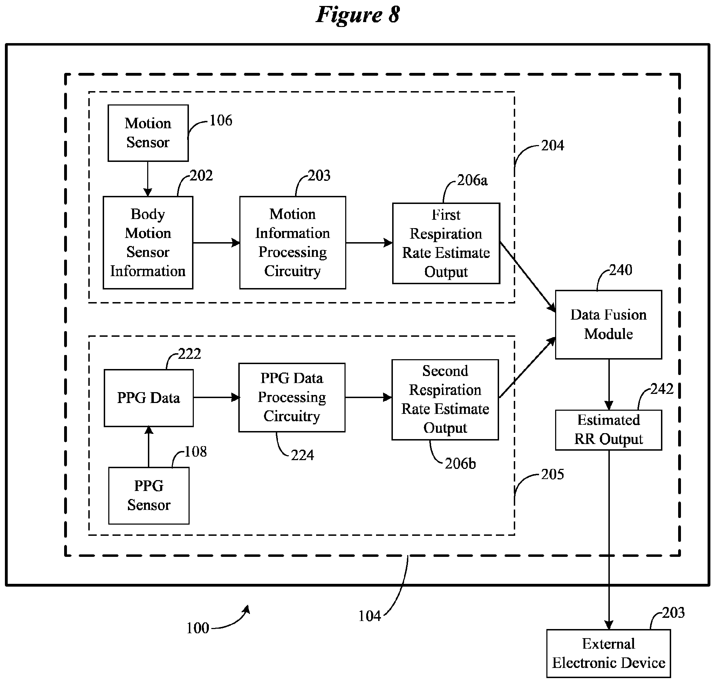

Figure 8 illustrates a respiration sensor 104 deployed in an ear-wearable electronic device 100 in accordance with any of the embodiments disclosed herein. The respiration sensor 104 shown in Figure 8 includes motion sensing circuitry 204 and PPG sensing circuitry 205. The components and functionality of the motion sensing circuitry 204 are equivalent to that described previously with reference to Figure 2. The components and functionality of the PPG sensing circuitry 205 are equivalent to that described previously with reference to Figure 5. The motion sensing circuitry 204 produces a first respiration rate estimate output 206a which is communicated to a data fusion module 240. The PPG sensing circuitry 205 produces a second respiration rate estimate output 206b which is communicated to the data fusion module 240. The data fusion module 240 is configured to process the first and second respiration rate outputs 206a, 206b to produce an estimated respiration output 242 (see, e.g., Figure 10). The estimated respiration output 242 can be stored internally within the memory 132 of the ear-wearable electronic device 100 and/or communicated to an external electronic device 208.

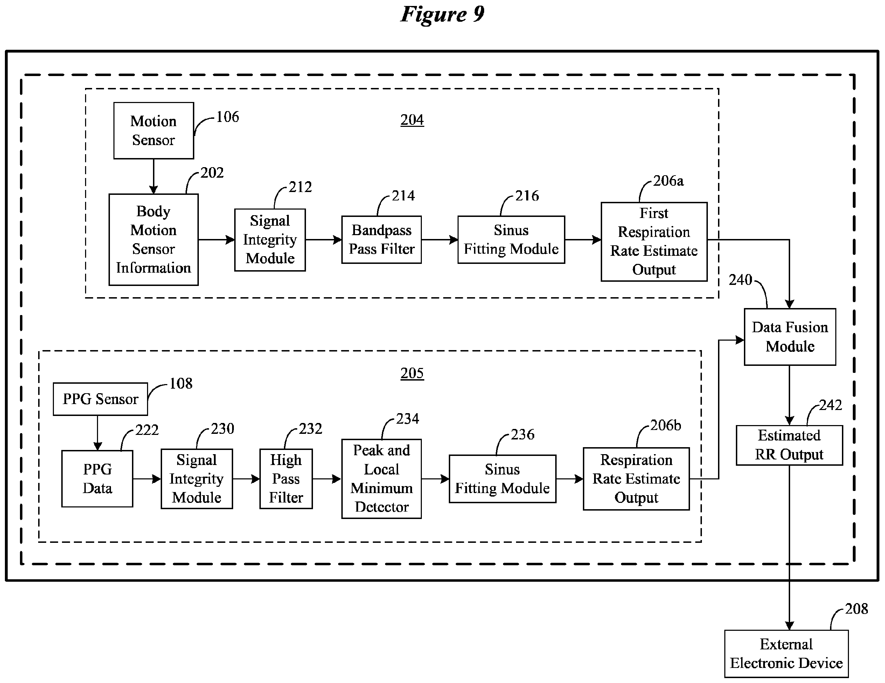

Figure 9 illustrates a respiration sensor 104 deployed in an ear-wearable electronic device 100 in accordance with any of the embodiments disclosed herein. Respiration sensor 104 shown in Figure 9 includes motion sensing circuitry 204 and PPG sensing circuitry 205. The components and functionality of the motion sensing circuitry 204 are equivalent to that described previously with reference to Figure 4. The components and functionality of the PPG sensing circuitry 205 are equivalent to that previously described with reference to Figure 7. The motion sensing circuitry 204 produces a first respiration rate estimate output 206a which is communicated to a data fusion module 240. The PPG sensing circuitry 205 produces a second respiration rate estimate output 206b which is communicated to the data fusion module 240. The data fusion module 240 is configured to process the first and second respiration rate outputs 206a, 206b to produce an estimated respiration output 242. The estimated respiration output 242 can be stored internally within the memory 132 of the ear-wearable electronic device 100 and/or communicated to an external electronic device 208.

Figure 10 illustrates additional processing details implemented by the PPG sensing circuitry 205 and the motion sensing circuitry 204 shown in previous figures. Process flow A involves the processing of M seconds (e.g., 60 seconds) of PPG data 1000 by the PPG sensing circuitry 205. Process flow A also involves selection 1002 of the best (e.g., most useful or suitable) N seconds (e.g., 20 seconds) of the M seconds of PPG data 1000, the result of which is a time window 1004 of N seconds of PPG data. The N seconds of PPG data 1004 is applied to a high pass filter 1006 having a specified corner frequency (e.g., f_{corner} = 0.3 Hz). The locations and heights of the peaks in the high pass-filtered PPG data are identified, such as by using a peak and local minimum detector. Process flow A concludes by applying a sinus-fitting algorithm (e.g., least-squares fitting) on the heights of the peaks, from which an estimated respiration rate 1012 using the PPG data is computed.

Process flow B involves the processing of P seconds (e.g., 60 seconds) of motion sensor data 1020 by the motion sensing circuitry 204. Process flow B also involves selection 1022 of the best (e.g., most useful or suitable) Q seconds (e.g., 20 seconds) of the P seconds of motion sensor data 1020, the result of which is a time window 1024 of Q seconds of motion data. The Q seconds of motion data 1024 is applied to a bandpass filter 1026 having a specified passband (e.g., from about 0.15 Hz to about 0.5 Hz). Process flow B concludes by applying a sinus-fitting algorithm (e.g., least-squares fitting) on the bandpass-filtered motion sensor data, from which an estimated respiration rate 1030 using the motion sensor data is computed.

Data fusion is performed on the estimated respiration rates 1012, 1030 to determine the output estimated respiration rate generated by the respiration sensor 104. Process flow C involves fusing 1040 the estimated respiration rate 1012 using the PPG data and the estimated respiration rate 1030 using the motion sensor to generate 1042 an output estimated respiration rate. According to some implementations, process flow C can involve a comparison of the estimated respiration rate using the PPG data to a threshold (e.g., 25). If the estimated respiration rate using the PPG data exceeds the threshold, the estimated respiration rate using the PPG sensor is output by the respiration sensor 104. If the estimated respiration rate using the PPG data does not exceed the threshold, the estimated respiration rate using the motion sensor is output by the respiration sensor 104. In other implementations, a fusing the estimated respiration rates 1012 and 1030 can involve weighting each of the estimated respiration rate 1012 using the PPG data and the estimated respiration rate 1030 using the motion sensor to generate 1042 an output estimated respiration rate. Weighting can be based on a number of factors, including, for example, the availability and/or relative integrity of the PPG and motion sensor data.

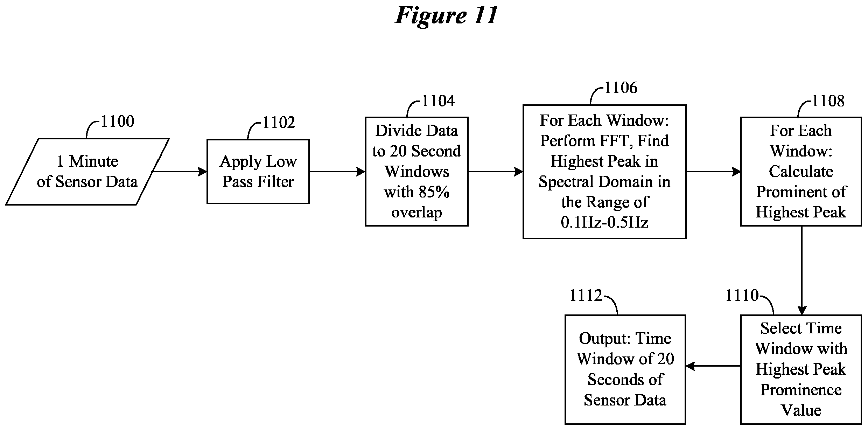

Figure 11 illustrates a method of selecting the best (e.g., most useful or suitable) window of time from a sampling of PPG data and/or motion sensor data in accordance with any of the embodiments disclosed herein. In the representative examples shown in Figure 11, one minute of sensor data is acquired 1100. It is understood that the amount of sensor data acquired in block 1100 can be longer or shorter than one minute. This sensor data is applied 1102 to a low pass filter having a specified corner frequency (e.g., f_{corner} = 0.5 Hz). The method involves dividing 1104 the low pass-filtered sensor data into 20 second windows with an 85% overlap. It is understood that the amount of low-pass filtered sensor data acquired in block 1104 and the extent of the overlap can be longer or shorter than shown in Figure 11.

In block 1106, for each of the 20 second windows, a fast Fourier transform (FFT) is performed on the data, and the highest peak in the spectral domain in the range of 0.1 Hz to 0.5 Hz is determined. In block 1108, for each of the 20 second windows, the prominence of the highest peak is calculated. The time window having the highest peak prominence value is selected 1110, resulting in the output 1112 of the selected time window of 20 seconds of sensor data.

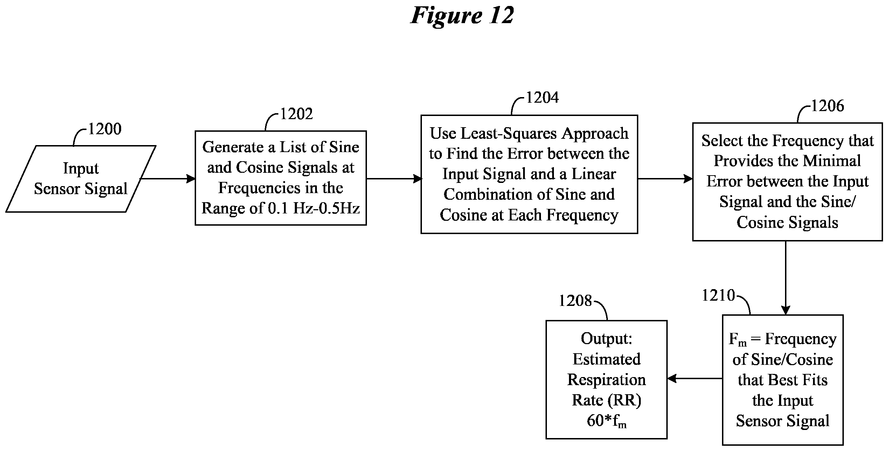

Figure 12 illustrates a method of applying a sinus-fitting algorithm on PPG data and/or motion sensor data in accordance with any of the embodiments disclosed herein. The method shown in Figure 12 begins with the input 1200 of a PPG or motion sensor signal, from which a list of sine and cosine signals at frequencies in the range of 0.1 Hz to 0.5 Hz is generated 1202. The method involves using 1204 a least-squares approach to find the error between the input signal and a linear combination of sine and cosine at each of the frequencies. The method also involves selecting 1206 the frequency that provides the minimal error between the input sensor signal and the sine/cosine signals. This results in selection 1210 of the frequency, Fₘ, of the sine/cosine list that best fits the input sensor signal. The method further involves the output 1208 of the estimated respiration rate using the equation RR = 60*fₘ.

Figure 13 illustrates a method for activating a respiration rate sensor of an ear-wearable electronic device in accordance with any of the embodiments disclosed herein. The method shown in Figure 13 involves criteria for respiration rate algorithm activation. The method shown in Figure 13 involves performing motion sensor and PPG sensor processing 1300, which involves activating the sensors and receiving sensor signals by a processor of the ear-wearable electronic device 100. The processing of motion and PPG sensor signals by the processor involves performing a signal integrity test. The signal integrity test involves the integrity of the motion and PPG sensor signals based on various calculations such as signal to noise ratio, skin contact integrity (e.g., resistance, perfusion index), and/or a correlation coefficient, R, for example.

If the signal integrity test is negative, as tested at block 1302, processing of motion and PPG sensor signals continues at block 1300. If the signal integrity test is positive, an activity status test is initiated. The activity status test can involve determining whether high respiration rate, if it exists, is caused by the wearer's current or prior activity. To be negative, both prior activity (e.g., in the prior 15-minutes period) and current activity need to be negative. For example, the activity status test can involve sensing of the wearer's level of physical activity using the motion sensor, and determining if the sensed level of physical activity is consistent with the wearer's respiration rate. Increased physical activity is associated with increased respiration rate, while decreased physical activity is associated with decreased respiration rate (e.g., within certain ranges). If the measure of physical activity is out of synchrony with the respiration rate (e.g., relative to predetermined ranges), then the activity status test may return a positive result.

If the activity status test is positive, as tested at block 1304, processing of motion and PPG sensor signals continues at block 1300. If the activity status test is negative, a respiration data validity test is initiated. The respiration rate validity (RRV) is calculated by the respiration algorithm implemented by the processor, based on the spectral content of the respiration rate signal. Criteria of the RRV calculation involves summing the energy level in all frequencies relevant for the RR estimation in the spectral domain. If the respiration rate validity test is not positive (e.g., negative), as tested at block 1306, processing of motion and PPG sensor signals continues at block 1300. If the respiration rate validity test is positive, processing continues at block 1308.

Assuming that all of the tests referenced in blocks 1302, 1304, and 1306 have been satisfied, the method continues by calculating the estimated respiration rate at block 1308. In some embodiments, the respiration rate data is communicated 1310 to a cloud database. It is noted that other physiologic data can be communicated to the cloud database, including heart rate, SpO2, temperature, electrodermal/skin contact data, and biochemical data (see physiologic sensors and parameters/conditions discussed with reference to Figure 1.) A cloud processor can be configured to calculate 1312 an early warning score (e.g., poor respiration score relative to an acceptable respiration score) using the respiration rate data, perform long-term analysis (trends analyses), and generate various diagnostic reports. This and other data produced by the cloud processor can be communicated 1314 to the wearer or the wearer's caregiver via an external electronic device (e.g., a smartphone, tablet, laptop). For example, the wearer and/or the caregiver can receive the wearer's daily average respiration rate and standard deviation in respiration rate. Long-term (e.g., trending) data analyses can include tracking changes in the wearer's respiration rate, and can also include correlating such changes with other measurable parameters (e.g., activity (e.g., before the measurement), human interaction, location, mood, etc.).

It is understood that, rather than or in addition to sending respiration data to the cloud database at block 1310, calculation of the early warning score, long-term analysis of the respiration data, and generation of diagnostic reports can be performed by an external electronic device operated by the wearer or the wearer's caregiver. In some implementations, an on-request (e.g. discretional) measurement can be initialized 1316 using an external electronic device operated by the wearer or the wearer's caregiver.

The method shown in Figure 13 can be performed on a regular basis (e.g., hourly, every 4 hours, every 8 hours) and/or on a commanded on-request basis. For example, the operations shown in blocks 1300, 1302, 1304, and 1306 can be performed on a cyclical basis each N hours (e.g., N = 1 or 2 hours). If, after M hours (e.g., M = 8), the method does not progress to block 1308, the respiration rate sensor can be switched to a continuous mode of operation, and an alert can be generated. The alert can be communicated to an external electronic device, such as a smartphone operated by the wearer of the ear-wearable electronic device 100 or a caregiver (e.g., via the cloud). In addition, or alternatively, the alert can be communicated from the receiver or speaker of the ear-wearable electronic device 100 to the wearer's ear drum.

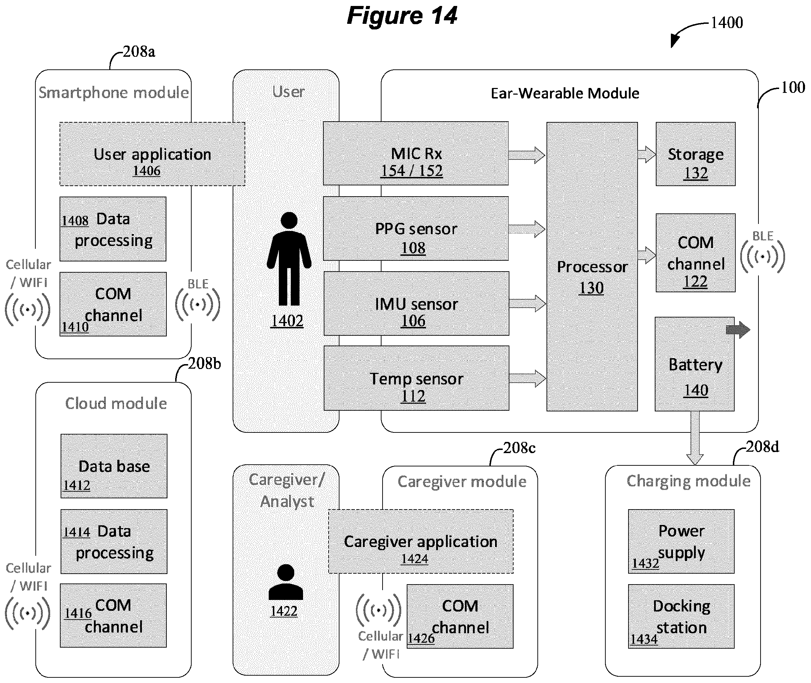

Figure 14 illustrates a system involving the generation of respiration rate data by an ear-wearable electronic device in accordance with any of the embodiments disclosed herein. The system 1400 shown in Figure 14 can be used implement the method shown in Figure 13. The system 1400 includes the previously-described ear-wearable module 100, a smartphone module 208a, a cloud module 208b, a caregiver module 208c, and a charging module 208d. The ear-wearable module 100 includes a number of components such as a processor 130, memory 132, a communication device 122, a rechargeable battery 140, one or more microphones 154 and a receiver/speaker 152, a PPG sensor 108, a motion sensor 106, and, in some embodiments, a temperature sensor 112 (and/or other physiologic sensors disclosed herein). The ear-wearable electronic device 100 communicates with the smartphone module 208a via a communication channel 122, such as a BLE link.

The wearer 1402 of the ear-wearable electronic device 100 interacts with the device 100 via a user application 1406 supported by a processor and user interface of the smartphone module 208a. Smart phone module 208a includes a data processing facility 1408 and a communication device 1410, which can communicate with the communication device 122 of the ear-wearable electronic device 100 and may also include a cellular and/or Wi-Fi radio(s). The cloud module 208b includes a cloud database 1412, a data processing facility 1414 (e.g., a cloud processor), and a communication device 1416, such as a cellular and/or Wi-Fi radio(s).

The caregiver module 208c is configured to implement a caregiver application 1424 and includes a communication device 1426 (e.g., cellular and/or Wi-Fi radio(s)) configured to communicate with the cloud module 208b. A caregiver/analyst 1422 can interact with the smartphone module 208a and ear-wearable electronic device 100 in a manner described with reference to Figure 13 (e.g., receiving and reviewing respiration rate data at block 1314, initiating an on-request measurement at block 1316). The charging module 208d includes a power supply 1432 and power management circuitry for charging the battery 140 of the ear-wearable electronic device 100. The charging module 208d can also include, or be configured as, a docking station 1434.

Although reference is made herein to the accompanying set of drawings that form part of this disclosure, one of at least ordinary skill in the art will appreciate that various adaptations and modifications of the embodiments described herein are within, or do not depart from, the scope of this disclosure. It is understood that the invention is defined by the appended claims.

Unless indicated to the contrary, the numerical parameters set forth in the foregoing specification and attached claims are approximations that can vary depending upon the desired properties sought to be obtained by those skilled in the art utilizing the teachings disclosed herein or, for example, within typical ranges of experimental error.

The recitation of numerical ranges by endpoints includes all numbers subsumed within that range (e.g. 1 to 5 includes 1, 1.5, 2, 2.75, 3, 3.80, 4, and 5) and any range within that range. Herein, the terms "up to" or "no greater than" a number (e.g., up to 50) includes the number (e.g., 50), and the term "no less than" a number (e.g., no less than 5) includes the number (e.g., 5).

The terms "coupled" or "connected" refer to elements being attached to each other either directly (in direct contact with each other) or indirectly (having one or more elements between and attaching the two elements). Either term may be modified by "operatively" and "operably," which may be used interchangeably, to describe that the coupling or connection is configured to allow the components to interact to carry out at least some functionality (for example, a radio chip may be operably coupled to an antenna element to provide a radio frequency electric signal for wireless communication).

Terms related to orientation, such as "top," "bottom," "side," and "end," are used to describe relative positions of components and are not meant to limit the orientation of the embodiments contemplated. For example, an embodiment described as having a "top" and "bottom" also encompasses embodiments thereof rotated in various directions unless the content clearly dictates otherwise.

Reference to "one embodiment," "an embodiment," "certain embodiments," or "some embodiments," etc., means that a particular feature, configuration, composition, or characteristic described in connection with the embodiment is included in at least one embodiment of the disclosure. Thus, the appearances of such phrases in various places throughout are not necessarily referring to the same embodiment of the disclosure. Furthermore, the particular features, configurations, compositions, or characteristics may be combined in any suitable manner in one or more embodiments.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the disclosure.

As used in this specification and the appended claims, the singular forms "a," "an," and "the" encompass embodiments having plural referents, unless the content clearly dictates otherwise. As used in this specification and the appended claims, the term "or" is generally employed in its sense including "and/or" unless the content clearly dictates otherwise.

As used herein, "have," "having," "include," "including," "comprise," "comprising" or the like are used in their open-ended sense, and generally mean "including, but not limited to." It will be understood that "consisting essentially of," "consisting of," and the like are subsumed in "comprising," and the like. The term "and/or" means one or all of the listed elements or a combination of at least two of the listed elements.

The phrases "at least one of," "comprises at least one of," and "one or more of" followed by a list refers to any one of the items in the list and any combination of two or more items in the list.

## Claims

1. A method of determining respiration rate using an ear-wearable electronic device (100), comprising:
obtaining (300) motion information (202) from a motion sensor (106) of the ear-wearable electronic device;
generating (302) a first respiration rate estimate (206a; 1030) using the motion information;
obtaining (600) photoplethysmographic, PPG, data (222) from a PPG sensor (108) of the ear-wearable electronic device;
generating (602) a second respiration rate estimate (206b; 1012) using the PPG data;
producing (1040) a respiration rate estimate using the first and second respiration rate estimates;
performing an activity status test (1304) using the motion information and PPG data; and
determining if a measure of the wearer's physical activity based on the motion information is consistent with the wearer's respiration rate estimated using the PPG data.

2. The method according to claim 1, wherein generating the first respiration rate estimate comprises:
filtering (1026) the motion information using a bandpass filter (214) configured to pass frequencies in a frequency range consistent with human breathing; and
applying (1028) a sinus fitting to the bandpass-filtered motion information to generate the first respiration rate estimate.

3. The method according to claim 1 or 2, wherein generating the second respiration rate estimate comprises:
filtering (1006) the PPG data using a high pass filter (232) having a specified cutoff frequency;
performing a time domain-to-frequency domain transform on the high pass-filtered PPG data;
performing (1008) peak and local minimum detection on the transformed PPG data; and
applying (1010) a sinus fitting to heights of peaks of the transformed PPG data to generate the second respiration rate estimate.

4. The method according to any one of claims 1 to 3, comprising:
capturing (1020) the motion information in a plurality of temporally spaced first windows;
capturing (1000) the PPG data in a plurality of temporally spaced second windows; and
selecting (1022; 1002) one of the first windows and one of the second windows for processing based on predefined spectral content criteria,
preferably wherein the predefined spectral content criteria comprises a highest peak in a spectral domain in the range of about 0.1 Hz to about 0.5 Hz.

5. The method according to any one of claims 1 to 4, comprising:
performing (1302) a test of motion sensor signal integrity; and
performing (1302) a test of PPG sensor signal integrity.

6. The method according to any one of claims 1 to 5, wherein producing the respiration rate estimate comprises processing the first and second respiration rate estimates using a fusion algorithm to produce the respiration rate estimate.

7. The method according to any one of claims 1 to 6, wherein producing the respiration rate estimate comprises:
comparing the second respiration rate estimate to a threshold; and
outputting the second respiration rate estimate in response to the second respiration rate estimate exceeding the threshold.

8. The method according to any one of claims 1 to 7, wherein producing the respiration rate estimate comprises:
comparing the second respiration rate estimate to a threshold; and
outputting the first respiration rate estimate in response to the second respiration rate estimate failing to exceed the threshold.

9. The method according to any one of claims 1 to 8, comprising performing a validity test (1306) of the second respiration rate estimate by comparing the second respiration rate estimate to a threshold.

10. The method according to any one of claims 1 to 9, comprising calculating the respiration rate estimate of the wearer in response to successful signal integrity (1302), activity (1304), and validity (1306) tests.

11. The method according to any one of claims 1 to 10, comprising communicating (1310, 1314) respiration rate data alone or in combination with other physiologic data to one or both of an external electronic device (208, 208a, 208c) e and a cloud database (1412).

12. The method according to claim 11, comprising generating (1312) one or more of an early warning score, long term analyses, and respiration rate trending reports by one or both of the external electronic device (208, 208a, 208c) or a cloud processor (1414).

13. An ear-wearable electronic device (100), comprising:
a motion sensor (106) configured to generate motion information (202) and motion information processing circuitry (203) configured to generate a first respiration rate estimate (206a; 1030) using the motion information;
a photoplethysmographic, PPG, sensor (108) configured to generate PPG data (222) and PPG data processing circuitry (224) configured to generate a second respiration rate estimate (206b; 1012) using the PPG data; and
a processor (130) configured to produce a respiration rate estimate using the first and second respiration rate estimates;
wherein the processor (130) is further configured to:
perform an activity status test (1304) using the motion information and PPG data; and
determine if a measure of the wearer's physical activity based on the motion information is consistent with the wearer's respiration rate estimated using the PPG data.

14. The device according to claim 13, wherein the device is adapted to perform a method according to any one of claims 2 to 12.

## Patentansprüche

1. Verfahren zur Atmungsratenbestimmung unter Verwendung einer Ohr-tragbaren elektronischen Vorrichtung (100), aufweisend:
Erhalten (300) von Bewegungsinformationen (202) von einem Bewegungssensor (106) der Ohr-tragbaren elektronischen Vorrichtung;
Erzeugen (302) einer ersten Atmungsratenschätzung (206a; 1030) unter Verwendung der Bewegungsinformationen;
Erhalten (600) photoplethysmographischer, PPG, Daten (222) von einem PPG-Sensor (108) der Ohr-tragbaren elektronischen Vorrichtung;
Erzeugen (602) einer zweiten Atmungsratenschätzung (206b; 1012) unter Verwendung der PPG-Daten;
Erzeugen (1040) einer Atmungsratenschätzung unter Verwendung der ersten und zweiten Atmungsratenschätzung;
Durchführen eines Aktivitätsstatustests (1304) unter Verwendung der Bewegungsinformationen und PPG-Daten; und
Bestimmen, ob ein auf den Bewegungsinformationen basierendes Maß der körperlichen Aktivität des Trägers konsistent ist mit der unter Verwendung der PPG-Daten geschätzten Atmungsrate des Trägers.

2. Verfahren nach Anspruch 1, wobei das Erzeugen der ersten Atmungsratenschätzung aufweist:
Filtern (1026) der Bewegungsinformationen unter Verwendung eines Bandpassfilters (214), der konfiguriert ist, Frequenzen in einem Frequenzbereich durchzulassen, der konsistent ist mit der menschlichen Atmung; und
Anwenden (1028) einer Sinusanpassung auf die bandpassgefilterten Bewegungsinformationen, um die erste Atmungsratenschätzung zu erzeugen.

3. Verfahren nach Anspruch 1 oder 2, wobei das Erzeugen der zweiten Atmungsratenschätzung aufweist:
Filtern (1006) der PPG-Daten unter Verwendung eines Hochpassfilters (232), der eine bestimmte Grenzfrequenz hat;
Durchführen einer Zeitdomäne-zur-Frequenzdomäne-Transformation an den hochpassgefilterten PPG-Daten;
Durchführen (1008) einer Spitzen- und lokalen Minimum-Detektion an den transformierten PPG-Daten; und
Anwenden (1010) einer Sinusanpassung an Höhen von Spitzen der transformierten PPG-Daten, um die zweite Atmungsratenschätzung zu erzeugen.

4. Verfahren nach irgendeinem der Ansprüche 1 bis 3, aufweisend:
Erfassen (1020) der Bewegungsinformationen in einer Mehrzahl von zeitlich beabstandeten ersten Fenstern;
Erfassen (1000) der PPG-Daten in einer Mehrzahl von zeitlich beabstandeten zweiten Fenstern; und
Auswählen (1022; 1002) eines der ersten Fenster und eines der zweiten Fenster zur Verarbeitung basierend auf vordefinierten spektralen Inhaltskriterien,
vorzugsweise wobei die vordefinierten spektralen Inhaltskriterien eine höchste Spitze in einer spektralen Domäne im Bereich von etwa 0,1 Hz bis etwa 0,5 Hz aufweisen.

5. Verfahren nach irgendeinem der Ansprüche 1 bis 4, aufweisend:
Durchführen (1302) eines Tests von Bewegungsensor-Signalintegrität; und
Durchführen (1302) eines Tests von PPG-Sensor-Signalintegrität.

6. Verfahren nach irgendeinem der Ansprüche 1 bis 5, wobei das Erzeugen der Atmungsratenschätzung ein Verarbeiten der ersten und zweiten Atmungsratenschätzung unter Verwendung eines Fusionsalgorithmus aufweist, um die Atmungsratenschätzung zu erzeugen.

7. Verfahren nach irgendeinem der Ansprüche 1 bis 6, wobei das Erzeugen der Atmungsratenschätzung aufweist:
Vergleichen der zweiten Atmungsratenschätzung mit einem Schwellenwert; und
Ausgeben der zweiten Atmungsratenschätzung als Reaktion darauf, dass die zweite Atmungsratenschätzung den Schwellenwert überschreitet.

8. Verfahren nach irgendeinem der Ansprüche 1 bis 7, wobei das Erzeugen der Atmungsratenschätzung aufweist:
Vergleichen der zweiten Atmungsratenschätzung mit einem Schwellenwert; und
Ausgeben der ersten Atmungsratenschätzung als Reaktion darauf, dass die zweite Atmungsratenschätzung den Schwellenwert nicht überschreitet.

9. Verfahren nach irgendeinem der Ansprüche 1 bis 8, aufweisend ein Durchführen eines Validitätstests (1306) der zweiten Atmungsratenschätzung durch Vergleichen der zweiten Atmungsratenschätzung mit einem Schwellenwert.

10. Verfahren nach irgendeinem der Ansprüche 1 bis 9, aufweisend ein Berechnen der Atmungsratenschätzung des Trägers als Reaktion auf erfolgreiche Signalintegritäts- (1302), Aktivitäts- (1304) und Validitäts- (1306) Tests.

11. Verfahren nach irgendeinem der Ansprüche 1 bis 10, aufweisend ein Übermitteln (1310, 1314) von Atmungsratendaten allein oder in Kombination mit anderen physiologischen Daten an eine oder beide aus einer externen elektronischen Vorrichtung (208, 208a, 208c) und einer Cloud-Datenbank (1412).

12. Verfahren nach Anspruch 11, aufweisend ein Erzeugen (1312) von einem oder mehreren aus einem Frühwarnwert, Langzeitanalysen, und Atmungsraten-Trendberichten durch eine oder beide aus der externen elektronischen Vorrichtung (208, 208a, 208c) oder einem Cloud-Prozessors (1414).

13. Ohr-tragbare elektronische Vorrichtung (100), aufweisend:
einen Bewegungssensor (106), der konfiguriert ist, Bewegungsinformationen (202) zu erzeugen, und eine Bewegungsinformationsverarbeitungsschaltung (203), die konfiguriert ist, unter Verwendung der Bewegungsinformationen eine erste Atmungsratenschätzung (206a; 1030) zu erzeugen;
einen photoplethysmographischen, PPG, Sensor (108), der konfiguriert ist, PPG-Daten (222) zu erzeugen, und eine PPG-Daten-Verarbeitungsschaltung (224), die konfiguriert ist, eine zweite Atmungsratenschätzung (206b; 1012) unter Verwendung der PPG-Daten zu erzeugen; und
einen Prozessor (130), der konfiguriert ist, eine Atmungsratenschätzung unter Verwendung der ersten und zweiten Atmungsratenschätzung zu erzeugen;
wobei der Prozessor (130) ferner konfiguriert ist, um:
einen Aktivitätsstatus-Test (1304) unter Verwendung der Bewegungsinformationen und PPG-Daten durchzuführen; und
zu bestimmen, ob ein auf den Bewegungsinformationen basierendes Maß der körperlichen Aktivität des Trägers konsistent ist mit der unter Verwendung der PPG-Daten geschätzten Atmungsrate des Trägers.

14. Vorrichtung nach Anspruch 13, wobei die Vorrichtung eingerichtet ist, ein Verfahren nach irgendeinem der Ansprüche 2 bis 12 durchzuführen.

## Revendications

1. Procédé de détermination du rythme respiratoire en utilisant un dispositif électronique à porter à l'oreille (100), comprenant les étapes suivantes :
obtenir (300) des informations de mouvement (202) à partir d'un capteur de mouvement (106) du dispositif électronique à porter à l'oreille ;
générer (302) une première estimation de rythme respiratoire (206a ; 1030) en utilisant les informations de mouvement ;
obtenir (600) des données photopléthysmographiques, PPG, (222) à partir d'un capteur PPG (108) du dispositif électronique à porter à l'oreille ;
générer (602) une deuxième estimation de rythme respiratoire (206b ; 1012) en utilisant les données PPG ;
produire (1040) une estimation de rythme respiratoire en utilisant les première et deuxième estimations de rythme respiratoire ;
réaliser un test de statut d'activité (1304) en utilisant les informations de mouvement et les données PPG ; et
déterminer si une mesure de l'activité physique du porteur basée sur les informations de mouvement est cohérente avec le rythme respiratoire du porteur estimé en utilisant les données PPG.

2. Procédé selon la revendication 1, dans lequel la génération de la première estimation de rythme respiratoire comprend :
le filtrage (1026) des informations de mouvement en utilisant un filtre passe-bande (214) configuré pour laisser passer les fréquences dans une plage de fréquences cohérente avec la respiration humaine ; et
l'application (1028) d'un ajustement sinusoïdal aux informations de mouvement filtrées par passe-bande pour générer la première estimation de rythme respiratoire.

3. Procédé selon la revendication 1 ou 2, dans lequel la génération de la deuxième estimation de rythme respiratoire comprend :
le filtrage (1006) des données PPG en utilisant un filtre passe-haut (232) ayant une fréquence de coupure spécifiée ;
la réalisation d'une transformation du domaine temporel vers le domaine fréquentiel sur les données PPG filtrées par passe-haut ;
la réalisation (1008) d'une détection des pics et des minima locaux sur les données PPG transformées ; et
l'application (1010) d'un ajustement sinusoïdal à des hauteurs de pics des données PPG transformées pour générer la deuxième estimation de rythme respiratoire.

4. Procédé selon l'une quelconque des revendications 1 à 3, comprenant les étapes suivantes :
capturer (1020) les informations de mouvement dans une pluralité de premières fenêtres espacées dans le temps ;
capturer (1000) les données PPG dans une pluralité de deuxièmes fenêtres espacées dans le temps ; et
sélectionner (1022 ; 1002) une des premières fenêtres et une des deuxièmes fenêtres pour le traitement sur la base de critères de contenu spectral prédéfinis,
de préférence dans lequel les critères de contenu spectral prédéfinis comprennent le pic le plus haut dans un domaine spectral dans la plage d'environ 0,1 Hz à environ 0,5 Hz.

5. Procédé selon l'une quelconque des revendications 1 à 4, comprenant les étapes suivantes :
réaliser (1302) un test d'intégrité de signal de capteur de mouvement ; et
réaliser (1302) un test d'intégrité de signal de capteur PPG.

6. Procédé selon l'une quelconque des revendications 1 à 5, dans lequel la production de l'estimation de rythme respiratoire comprend le traitement des première et deuxième estimations de rythme respiratoire en utilisant un algorithme de fusion pour produire l'estimation de rythme respiratoire.

7. Procédé selon l'une quelconque des revendications 1 à 6, dans lequel la production de l'estimation de rythme respiratoire comprend :
la comparaison de la deuxième estimation de rythme respiratoire à un seuil ; et
la sortie de la deuxième estimation de rythme respiratoire en réponse au fait que la deuxième estimation de rythme respiratoire dépasse le seuil.

8. Procédé selon l'une quelconque des revendications 1 à 7, dans lequel la production de l'estimation de rythme respiratoire comprend :
la comparaison de la deuxième estimation de rythme respiratoire à un seuil ; et
la sortie de la première estimation de rythme respiratoire en réponse au fait que la deuxième estimation de rythme respiratoire ne dépasse pas le seuil.

9. Procédé selon l'une quelconque des revendications 1 à 8, comprenant la réalisation d'un test de validité (1306) de la deuxième estimation de rythme respiratoire en comparant la deuxième estimation de rythme respiratoire à un seuil.

10. Procédé selon l'une quelconque des revendications 1 à 9, comprenant le calcul de l'estimation de rythme respiratoire du porteur en réponse à la réussite des tests d'intégrité de signal (1302), d'activité (1304) et de validité (1306).

11. Procédé selon l'une quelconque des revendications 1 à 10, comprenant la communication (1310, 1314) de données de rythme respiratoire seules ou en combinaison avec d'autres données physiologiques à un dispositif électronique externe (208, 208a, 208c), à une base de données en nuage (1412), ou aux deux.

12. Procédé selon la revendication 11 comprenant la génération (1312) d'un ou plusieurs parmi un score d'alerte précoce, des analyses à long terme et des rapports de tendances de rythme respiratoire par le dispositif électronique externe (208, 208a, 208c), un processeur en nuage (1414) ou les deux.

13. Dispositif électronique à porter à l'oreille (100), comprenant :
un capteur de mouvement (106) configuré pour générer des informations de mouvement (202), et un ensemble de circuits de traitement d'informations de mouvement (203) configuré pour générer une première estimation de rythme respiratoire (206a ; 1030) en utilisant les informations de mouvement ;
un capteur photopléthysmographique, PPG, (108) configuré pour générer des données PPG (222), et un ensemble de circuits de traitement de données PPG (224) configuré pour générer une deuxième estimation de rythme respiratoire (206b ; 1012) en utilisant les données PPG ; et
un processeur (130) configuré pour produire une estimation de rythme respiratoire en utilisant les première et deuxième estimations de rythme respiratoire ;
dans lequel le processeur (130) est en outre configuré pour :
réaliser un test de statut d'activité (1304) en utilisant les informations de mouvement et les données PPG ; et
déterminer si une mesure de l'activité physique du porteur basée sur les informations de mouvement est cohérente avec le rythme respiratoire du porteur estimé en utilisant les données PPG.

14. Dispositif selon la revendication 13, dans lequel le dispositif est adapté pour mettre en œuvre un procédé selon l'une quelconque des revendications 2 à 12.
